# EUROPÄISCHE PATENTSCHRIFT

(11) **EP 2 124 802 B1**
(45) Veröffentlichungstag und Bekanntmachung des Hinweises auf die Patenterteilung: **23.08.2017**
(21) Anmeldenummer: 08717128.6
(22) Anmeldetag: 26.02.2008
(51) Int. Cl.: A61B 6/12, A61B 5/06, A61B 90/00, A61B 18/14, A61B 6/00, A61B 5/042

(54) **VERFAHREN UND VORRICHTUNG ZUR VISUELLEN UNTERSTÜTZUNG EINER KATHETERANWENDUNG**
METHOD AND DEVICE FOR VISUALLY ASSISTING A CATHETER APPLICATION
PROCÉDÉ ET SYSTÈME D'ASSISTANCE VISUELLE POUR L'APPLICATION D'UN CATHÉTER

(30) Priorität: 27.02.2007 DE 102007009764
(43) Veröffentlichungstag der Anmeldung: 02.12.2009
(73) Patentinhaber: Biosense Webster, Inc., Diamond Bar, CA 91765 (US)
(72) Erfinder: BOESE, Jan, 90542 Eckental (DE); JOHN, Matthias, 90429 Nürnberg (DE); RAHN, Norbert, 91301 Forchheim (DE); BARTAL, Meir, 32240 Haifa (IL); GOVARI, Assaf, 34400 Haifa (IL); PREISS, Assaf, 17906 Shimshit (IL)
(74) Vertreter: Schohe, Stefan
(86) Internationale Anmeldenummer: PCT/EP2008/052295
(87) Internationale Veröffentlichungsnummer: WO 2008/104530

(56) Entgegenhaltungen:
- WO-A-2005/111942
- DE-A1- 10 340 546
- US-A1- 2003 139 670
- US-A1- 2004 097 805
- US-A1- 2004 215 071
- US-A1- 2005 033 149
- US-A1- 2005 251 028
- US-A1- 2005 281 385
- US-A1- 2006 116 575

## Beschreibung

Die Erfindung betrifft ein Verfahren und eine Vorrichtung zur visuellen Unterstützung einer Katheteranwendung am Herzen eines Patienten anhand von wenigstens einem mit einem C-Bogen-Röntgengerät von dem Patienten gewonnenen Bild und anhand von mit einem elektromagnetischen Positionserfassungs- und Mappingsystem gewonnenen elektroanatomischen Mapping-Daten von dem Patienten.

In verschiedenen Anwendungsbereichen der Medizintechnik ist es heutzutage üblich ein medizinisches Instrument, beispielsweise eine Punktionsnadel oder einen Katheter, in einen Patienten mit Hilfe von mit Bildgebungsgeräten zur Verfügung gestellten Bildinformationen zielgerichtet einzuführen, um den Patienten bzw. ein Gewebe oder ein Organ des Patienten mit dem Instrument zu untersuchen oder zu behandeln.

So werden beispielsweise Herzrhythmusstörungen eines Patienten durch eine sogenannte Ablation behandelt, bei der unter Röntgenkontrolle, beispielsweise basierend auf Röntgendurchleuchtungsbildern ein Ablationskatheter über Venen oder Arterien in eine der Herzkammern des Herzens des Patienten eingeführt und durch Hochfrequenzstrom das die Herzrhythmusstörungen hervorrufende Gewebe verödet wird. Voraussetzung für eine erfolgreiche Durchführung einer Katheterablation ist die genaue Ortung der Ursache der Herzrhythmusstörungen in der Herzkammer. Diese Ortung erfolgt über eine elektrophysiologische Untersuchung, bei der elektrische Potentiale mit einem in die Herzkammer eingeführten Mapping-Katheter ortsaufgelöst erfasst werden. Aus dieser elektrophysiologischen Untersuchung, dem so genannten elektroanatomischen Mapping, werden somit beispielsweise 3D-Mapping-Daten erhalten, die an einem Sichtgerät visualisiert werden können. Die Mapping-Funktion und die Ablations-Funktion sind häufig in einem Katheter vereint, so dass der Mapping-Katheter gleichzeitig auch ein Ablationskatheter ist.

Ein bekanntes elektroanatomisches 3D-Mapping-Verfahren, wie es z. B. mit dem CARTO-System der Fa. Biosense Webster, Inc., USA, durchführbar ist, basiert auf elektromagnetischen Prinzipien. Mit unter einer Patientenlagerungsvorrichtung angeordneten Transmittern werden in der Regel drei verschiedene elektromagnetische Wechselfelder geringer Intensität aufgebaut. Mittels in die Katheterspitze des Mapping-Katheters integrierter elektromagnetischer Sensoren ist es dann möglich, die durch Katheterbewegungen induzierten Spannungsänderungen innerhalb der elektromagnetischen Wechselfelder zu messen und mit Hilfe mathematischer Algorithmen zu jedem Zeitpunkt die Position des Mapping-Katheters zu errechnen. Durch punktweises Abtasten der endokardialen Kontur einer Herzkammer mit dem Mapping-Katheter bei simultaner Erfassung der elektrischen Signale der Sensoren erhält man 3D-Mapping-Daten bzw. entsteht eine elektroanatomische dreidimensionale Landkarte, in der die elektrischen Signale farbkodiert wiedergegeben werden.

Die Führung des Ablationskatheters kann daher nicht nur mit Hilfe der bereits erwähnten Röntgendurchleuchtungsbilder, sondern auch anhand der elektroanatomischen Mapping-Daten erfolgen, die beispielsweise mit dem bereits erwähnten CARTO-System der Fa. Biosense Webster Inc., USA, in Realtime erzeugt und auf einem Sichtgerät dargestellt werden können. Gerade die Durchleuchtungsbilder zeigen nämlich die Anatomie des Patienten, insbesondere die Anatomie des Herzens des Patienten nicht im Detail. Eine 3D-Darstellung anatomischer Details des Herzens könnte die Genauigkeit bei der Durchführung der Ablation in Bezug auf die Morphologie des Herzgewebes erhöhen, die Durchführung der Ablation beschleunigen und zu einer Reduzierung der einem Patienten während einer Ablation applizierten Röntgenstrahlendosis führen.

Insbesondere bei komplexen Fällen begrüßen es Elektrophysiologen, die Ablation anhand einer Kombination von elektrophysiologischen und morphologischen Kriterien durchfuhren zu können. Für die Elektrophysiologen wäre es daher hilfreich, eine kombinierte Visualisierung von mit einem Bildgebungsgerät gewonnenen 3D-Bilddaten und elektroanatomischen 3D-Mapping-Daten zur Verfugung zu haben.

In der DE 103 40 544 A1 und der DE 103 40 546 A1 sind beispielsweise Verfahren zur visuellen Unterstützung einer elektrophysiologischen Katheteranwendung beschrieben, bei denen vor der Durchführung der Katheteranwendung mit einem Verfahren der topographischen 3D-Bildgebung 3D-Bilddaten eines zu behandelnden Bereiches eines Patienten erfasst werden, wobei aus den 3D-Bilddaten durch Segmentierung ein 3D-Oberflächenverlauf von Objekten in dem zu behandelnden Bereich extrahiert wird und wobei anschließend bereitgestellte elektroanatomische 3D-Mapping-Daten und den 3D-Oberflächenverlauf bildende 3D-Bilddaten einander lage- und dimensionsrichtig zugeordnet und z.B. während der Durchführung der Katheteranwendung einander überlagert visualisiert werden. Diese Überlagerung von vor der Katheteranwendung beispielsweise mittels Computertomographie oder Magnetresonanztomographie gewonnenen 3D-Bilddaten von dem Patienten und elektroanatomischen 3D-Mapping-Daten des Patienten erfordert zuweilen eine zeitaufwändige und fehleranfällige markenbasierte oder oberflächenbasierte Registrierung der Daten miteinander, wobei sich Fehler bei der Registrierung negativ auf die Qualität und die Zuverlässigkeit von fusionierte Daten aufweisenden Bilder auswirken können. Die Genauigkeit der Registrierung beruht dabei auch auf der Anzahl von Oberflächenpunkten, welche mit dem Mapping-System gewonnen wurden.
US-A-2004/0215071 betrifft ein Verfahren und eine Vorrichtung einer 2D-zu-3D-Einschreibung. Ein dreidimensionales Bild aus einem dreidimensionalen Scan, wie z.B. einem CT-Scan, wird in ein zweidimensionales Fluoroskopiebild aus einer bildgebenden Vorrichtung eingeschrieben. Die Einschreibung ist der Vorgang zum Bestimmen, wie die Position des Instruments auf diagnostischem, vorher aufgenommenen oder Echtzeit-Bildern zu korrigieren ist. Das Verhältnis zwischen Sätzen von Punkten wird analysiert und eine Übereinstimmung wird berechnet, die jeden Punkt in jedem Satz mit dem entsprechenden Punkt in dem anderen Satz korreliert.

WO-A-2005/111942 betrifft ein medizinisches bildgebendes System zum Abbilden einer Struktur in einem Körper eines Patienten. Mehrere dreidimensionale Bilddatensätze werden erlangt. Jeder von mehreren Punkten eines medizinischen Instruments wird mit einem der dreidimensionalen Bilddatensätze verknüpft. Umwandlungen werden für jeden der dreidimensionalen Bilddatensätze zu einem dreidimensionalen Referenzbilddatensatz berechnet. Jeder Punkt wird dann durch Anwenden der Umwandlung des zugeordneten dreidimensionalen Bilddatensatzes umgewandelt. US2005/0251028 offenbart ein System zur gleichzeitigen Darstellung von Mapping-Daten und C-Bogen Röntgenbildern.

Der Erfindung liegt daher die Aufgabe zugrunde, ein Verfahren und eine Vorrichtung der eingangs genannten Art derart anzugeben, dass die kombinierte Anwendung von Bilddaten und Mapping-Daten vereinfacht wird.

Nach der Erfindung wird diese Aufgabe gelöst durch ein Verfahren und eine Vorrichtung zur visuellen Unterstützung einer Katheteranwendung am Herzen eines Patienten anhand von wenigstens einem mit einem C-Bogen-Röntgengerät von dem Patienten gewonnenen Bild und anhand von mit einem elektromagnetischen Positionserfassungs- und Mappingsystem gewonnenen elektroanatomischen Mapping-Daten von dem Patienten. Das wenigstens eine Bild von dem Patienten und die elektroanatomischen Mapping-Daten von den Patienten werden miteinander fusioniert oder einander überlagert. Das C-Bogen-Röntgengerät und das elektromagnetische Positionserfassungs- und Mappingsystem werden relativ zueinander kalibriert, indem eine Koordinatentransformation zwischen einem dem C-Bogen-Röntgengerät zugeordneten Koordinatensystem und/oder einem wenigstens einem mit dem C-Bogen-Röntgengerät erzeugten Bild zugeordneten Koordinatensystem und einem dem elektromagnetischen Positionserfassungs- und Mappingsystem zugeordneten Koordinatensystem ermittelt wird, und bei dem die Position des Patienten während der Erfassung des Bildes und während der Erfassung der elektroanatomischen Mapping-Daten, die vor der Katheteranwendung gewonnen wurden, ermittelt und dem Bild und/oder den elektroanatomischen Mapping-Daten zumindest indirekt zugeordnet wird.

Durch die Kalibrierung des C-Bogen-Röntgengerätes und des elektromagnetischen Positionserfassungs- und Mappingsystems relativ zueinander ist eine Registrierung von Bilddaten eines mit dem C-Bogen-Röntgengerät gewonnenen Bildes und von mit dem elektromagnetischen Positionserfassungs- und Mappingsystem gewonnenen Mapping-Daten während einer Katheteranwendung nicht mehr erforderlich, da die Transformationsbeziehung für die Daten der beiden Systeme bzw. Gerät nunmehr bekannt ist. Somit ist es unter der Vorrausetzung, dass der Patient seine Position zwischen und nach den Datenaufnahmen mit den beiden Systemen bzw. Geräten nicht verändert, möglich, Bilddaten von vor oder während einer Katheteranwendung mit dem C-Bogen-Röntgengerät gewonnenen Bildern von dem Patienten, seien es 2D-Bilder oder 3D-Bilder, und vor oder während der Katheteranwendung gewonnene Mapping-Daten, seien es 2D- oder 3D-Mapping-Daten, miteinander zu fusionieren oder einander zu überlagern, ohne eine zeitaufwändige oder fehleranfällige Registrierung der Daten vornehmen zu müssen. Dadurch dass zudem die Position des Patienten während der Erfassung des Bildes und/oder der Erfassung der elektroanatomischen Mapping-Daten ermittelt wird und dem Bild und/oder den elektroanatomischen Mapping-Daten zumindest indirekt zugeordnet wird, können darüber hinaus Positionsänderungen des Patienten nach der Erfassung des Bildes mit dem C-Bogen-Röntgengerät und der Erfassung der elektroanatomischen Mapping-Daten bei der Bildverarbeitung entsprechend berücksichtigt werden.

An dem C-Bogen-Röntgengerät, welches beispielsweise für Angiographieanwendungen vorgesehen ist, sind eine Röntgenstrahlenquelle und ein Röntgenstrahlenempfänger einander gegenüberliegend an einem C-Bogen angeordnet, welcher zur Aufnahme von 2D-Projektionen aus unterschiedlichen Projektionsrichtungen um den Patienten verstellt werden kann. Aus einer Serie von mit dem C-Bogen-Röntgengerät unter voneinander verschiedenen Projektionsrichtungen aufgenommenen 2D-Projektionen kann ein Volumendatensatz rekonstruiert werden. Aufgrund der bekannten Dimensionen des C-Bogen-Röntgengerätes und der bekannten Projektionsgeometrien der 2D-Projektionen ist auch die Transformationsbeziehung zwischen einem dem C-Bogen-Röntgengerät zugeordneten Koordinatensystem und einem dem Volumendatensatz oder einem aus dem Volumendatensatz erzeugten 3D-Bild zugeordneten Koordinatensystem bekannt.

Gemäß einer Variante der Erfindung ist dem C-Bogen-Röntgengerät eine Patientenlagerungsvorrichtung definiert zugeordnet, d.h. C-Bogen-Röntgengerät und Patientenlagerungsvorrichtung sind in bekannter Weise relativ zueinander angeordnet.

Nach einer weiteren Variante der Erfindung weist das elektromagnetische Positionserfassungs- und Mappingsystem wenigstens einen Transmitter zur Erzeugung eines elektromagnetischen Wechselfeldes und einen Katheter mit wenigstens einem Sensor auf. In der Regel weist das elektromagnetische Positionserfassungs- und Mappingsystem mehrere Transmitter, beispielsweise drei Transmitter, zur Erzeugung drei verschiedener Wechselfelder und der Katheter drei Sensoren auf, so dass mit Hilfe der drei in den Katheter integrierten Sensoren die Position des Katheters in einem dem elektromagnetischen Positionserfassungs- und Mappingsystem zugeordneten Koordinatensystem ermittelt werden kann.

Zur Ermittlung der Koordinatentransformation zwischen einem dem C-Bogen-Röntgengerät zugeordneten Koordinatensystem und/oder einem wenigstens einem mit dem C-Bogen-Röntgengerät erzeugten Bild zugeordneten Koordinatensystem und einem dem elektromagnetischen Positionserfassungs- und Mappingsystem zugeordneten Koordinatensystem wird der Transmitter nach einer Variante der Erfindung definiert an dem C-Bogen-Röntgengerät oder an der Patientenlagerungsvorrichtung angeordnet. Nach einer Ausführungsform der Erfindung wird der Transmitter definiert am C-Bogen des C-Bogen-Röntgengerätes angeordnet. Auf diese Weise kann eine feste Beziehung zwischen dem C-Bogen-Röntgengerät und dem elektromagnetischen Positionserfassungs- und Mappingsystem hergestellt werden.

Nach einer Ausführungsform der Erfindung ist der Transmitter von dem C-Bogen-Röntgengerät oder von der Patientenlagerungsvorrichtung abnehmbar. Sollte demnach der Transmitter beispielsweise bei der Aufnahme von Bildern von dem Patienten mit dem C-Bogen-Röntgengerät stören, so kann dieser während der Bildaufnahme von dem C-Bogen-Röntgengerät oder von der Patientenlagerungsvorrichtung abgenommen und anschließend wieder an der definierten Stelle an dem C-Bogen-Röntgengerät oder der Patientenlagerungsvorrichtung angeordnet werden.

Nach einer anderen Variante der Erfindung ist wenigstens ein Positions- und Orientierungssensor des elektromagnetischen Positionserfassungs- und Mappingsystems an oder in der Patientenlagerungsvorrichtung definiert angeordnet. In diesem Fall ist aufgrund der bekannten Beziehung zwischen dem C-Bogen-Röntgengerät und der Patientenlagerungsvorrichtung und der bekannten konstruktiven Anordnung des wenigstens einen Positions- und Orientierungssensor an oder in der Patientenlagerungsvorrichtung die Transformationsbeziehung zwischen dem dem C-Bogen-Röntgengerät zugeordneten Koordinatensystem und damit auch zwischen dem einem mit dem C-Bogen-Röntgengerät aufgenommenen Bild zugeordneten Koordinatensystem und dem dem elektromagnetischen Positionserfassungs- und Mappingsystem zugeordneten Koordinatensystem durch Erfassung des Positions- und Orientierungssensors mit dem elektromagnetischen Positionserfassungs- und Mappingsystem ermittelbar.

Nach einer Ausführungsform der Erfindung ist der wenigstens eine Positions- und Orientierungssensor des elektromagnetischen Positionserfassungs- und Mappingsystems von der Patientenlagerungsvorrichtung abnehmbar. Bevorzugt ist der Positions- und Orientierungssensor bzw. sind die Positions- und 0-rientierungssensoren in einem von der Patientenlagerungsvorrichtung abnehmbaren Modul oder auch in mehreren von der Patientenlagerungsvorrichtung abnehmbaren Modulen angeordnet, welche Module in definierter Weise relativ zueinander an der Patientenlagerungsvorrichtung angeordnet sind.

Nach einer Variante der Erfindung erfolgt die Ermittlung der Koordinatentransformation zwischen einem dem C-Bogen-Röntgengerät zugeordneten Koordinatensystem und/oder einem wenigstens einem mit dem C-Bogen-Röntgengerät erzeugten Bild zugeordneten Koordinatensystem und einem dem elektromagnetischen Positionserfassungs- und Mappingsystem zugeordneten Koordinatensystem anhand von wenigstens einer Marke, welche in einem erzeugten Bild abgebildet ist und mit dem elektromagnetischen Positionserfassungs- und Mappingsystem detektierbar ist. In der Regel werden mehrere Marken vorzugsweise mindestens drei Marken verwendet, um die Koordinatentransformation zu ermitteln. Dabei werden die Koordinaten der einzelnen Marken jeweils in den jeweiligen Koordinatensystemen bestimmt und anhand der bestimmten Koordinaten der jeweiligen Marken in den Koordinatensystemen die Koordinatentransformation zwischen den Koordinatensystemen ermittelt.

Bei der oder den Marken handelt sich um röntgenpositive Marken, beispielsweise kleine Metallkugeln, die in wenigstens zwei unter voneinander verschiedenen Projektionswinkeln aufgenommenen Röntgenprojektionen oder in einem 3D-Bild abgebildet werden. Nach Ausführungsformen der Erfindung können die Abbilder der Marken in den Projektionsbildern bzw. dem 3D-Bild manuell über eine graphische Benutzerschnittstelle oder automatisch mit einem Verfahren der Mustererkennung lokalisiert werden, so dass die für die Ermittlung der Koordinatentransformation erforderlichen Koordinaten der Marken in dem den Projektionsbildern oder dem 3D-Bildes zugeordneten Koordinatensystem durch Rückprojektion ermittelbar sind.

Für die Ermittlung der Koordinaten der Marken in dem dem elektromagnetischen Positionserfassungs- und Mappingsystem zugeordneten Koordinatensystem wird ein Positionssensor des elektromagnetischen Positionserfassungs- und Mappingsystems verwendet, mit dem die jeweiligen Marken berührt werden.

Nach einer Variante der Erfindung ist wenigstens eine Marke ein röntgenpositiver Positionssensor des elektromagnetischen Positionserfassungs- und Mappingsystems. Da die Marke selbst ein Positionssensor des elektromagnetischen Positionserfassungs- und Mappingsystems ist, muss diese zur Detektion und zur Bestimmung ihrer Koordinaten in dem dem elektromagnetischen Positionserfassungs- und Mappingsystem zugeordneten Koordinatensystem nicht eigens mit einem anderen Positionssensor berührt werden, sondern kann direkt und automatisch von dem elektromagnetischen Positionserfassungs- und Mappingsystem detektiert werden. Die Marke bzw. der röntgenpositive Positionssensor kann dabei eine Katheterspitze eines Katheters des elektromagnetischen Positionserfassungs- und Mappingsystems sein.

Nach einer Ausführungsform der Erfindung ist zur Ermittlung der Koordinatentransformation ein mit wenigstens einer Marke versehenes Phantom vorgesehen, welches in geeigneter Weise auf der Patientenlagerungsvorrichtung relativ zu dem C-Bogen-Röntgengerät und dem elektromagnetischen Positionserfassungs-und Mappingsystem zur Ermittlung der Koordinatentransformation angeordnet wird.

Nach einer Variante der Erfindung kann dabei wenigstens eine Marke des Phantoms ein Positionssensor des elektromagnetischen Positionserfassungs- und Mappingsystems sein, so dass, wie bereits erwähnt, die Marke nicht eigens mit einem Positionssensor berührt werden muss, sondern direkt und automatisch von dem elektromagnetischen Positionserfassungs- und Mappingsystem detektiert werden kann.

Nach einer Ausführungsform der Erfindung sind alle Marken des Phantoms Positionssensoren des elektromagnetischen Positionserfassungs- und Mappingsystems, so dass die Marken des Phantoms direkt und automatisch von dem elektromagnetischen Positionserfassungs- und Mappingsystem detektierbar und deren Koordinaten in dem dem elektromagnetischen Positionserfassungs-und Mappingsystem zugeordneten Koordinatensystem ermittelbar sind.

Nach einer anderen Variante der Erfindung weist das Phantom neben dem Positionssensor des elektromagnetischen Positionserfassungs- und Mappingsystems wenigstens eine, vorzugsweise mehrere weitere Marken auf, die keine Positionssensoren sind, wobei die Positionen bzw. Koordinaten der weiteren Marken des Phantoms relativ zu dem wenigstens einen Positionssensor des Phantoms bekannt sind. In diesem Fall genügt es den Positionssensor mit dem elektromagnetischen Positionserfassungs-und Mappingsystem automatisch zu detektieren und dessen Koordinaten zu ermitteln. Die Koordinaten der weiteren Marken in dem dem elektromagnetischen Positionserfassungs- und Mappingsystem zugeordneten Koordinatensystem ergeben sich dann aufgrund der bekannten Koordinaten der weiteren Marken relativ zu dem Positionssensor.

Nach einer Ausführungsform der Erfindung werden das mit Marken versehene Phantom und das elektromagnetische Positionserfassungs- und Mappingsystem derart definiert relativ zueinander angeordnet, dass die Koordinatentransformation zwischen einem dem Phantom zugeordneten Koordinatensystem und einem dem elektromagnetischen Positionserfassungs- und Mappingsystem zugeordneten Koordinatensystem bekannt ist. In diesem Fall müssen also die Koordinaten der Marken des Phantoms nicht eigens in dem dem Positionserfassungs- und Mappingsystem zugeordneten Koordinatensystem ermittelt werden. Vielmehr sind zur Berechnung der Koordinatentransformation zwischen dem dem C-Bogen-Röntgengerät zugeordneten Koordinatensystem bzw. dem einem mit dem C-Bogen-Röntgengerät gewonnenen Bild zugeordneten Koordinatensystem und dem dem elektromagnetischen Positionserfassungs- und Mappingsystem zugeordneten Koordinatensystem nur die Koordinaten der Marken des Phantoms in dem dem mit dem C-Bogen-Röntgengerät gewonnenen Bild zugeordneten Koordinatensystem zu ermitteln, um die Koordinatentransformation berechnen zu können.

Bei den bisher beschriebenen Verfahren zur Ermittlung der Koordinatentransformation zwischen dem dem C-Bogen-Röntgengerät zugeordneten Koordinatensystem bzw. dem einem mit dem C-Bogen-Röntgengerät gewonnenen Bild zugeordneten Koordinatensystem und dem dem elektromagnetischen Positionserfassungs-und Mappingsystem zugeordneten Koordinatensystem handelt es sich um so genannte offline-Verfahren, bei denen ein zu untersuchender Patient nicht anwesend ist.

Die Ermittlung der Koordinatentransformation kann jedoch auch online, d.h. unter Anwesenheit des Patienten erfolgen. Nach dieser Variante der Erfindung wird der Patient oder ein Teil oder ein Organ des Patienten mit Marken, vorzugsweise mit röntgenpositiven Marken, versehen, deren Abbilder in einem mit dem C-Bogen-Röntgengerät gewonnenen Bild lokalisiert und welche mit dem elektromagnetischen Positionserfassungs- und Mappingsystem detektierbar sind. Anschließend werden wieder die Koordinaten der Marken in dem dem Bild zugeordneten Koordinatensystemen und die Koordinaten der Marken in dem dem elektromagnetischen Positionserfassungs- und Mappingsystem zugeordneten Koordinatensystem ermittelt und basierend darauf die Koordinatentransformation zwischen den dem C-Bogen-Röntgengerät bzw. dem mit dem C-Bogen-Röntgengerät erzeugten Bild zugeordneten Koordinatensystem und dem dem elektromagnetischen Positionserfassungs- und Mappingsystem zugeordneten Koordinatensystem ermittelt.

Nach einer Variante der Erfindung wird die Position des Patienten kontinuierlich oder intermittierend während der Katheteranwendung ermittelt. Auf diese Weise ist die Position des Patienten stets bekannt, so dass Positionsänderungen des Patienten seit der Erfassung des Bildes mit dem C-Bogen-Röntgengerät und/oder der Erfassung der elektroanatomischen Mapping-Daten registriert und bei der Bildverarbeitung und Verwendung der Bilddaten entsprechend berücksichtigt werden können.

Nach einer Ausführungsform der Erfindung werden die Bilddaten des mit dem C-Bogen-Röntgengerät gewonnenen Bildes und die elektroanatomischen Mapping-Daten unter Berücksichtigung der Position des Patienten bei der Erfassung des Bildes und der elektroanatomischen Mapping-Daten miteinander fusioniert oder einander überlagert. Verändert also beispielsweise der Patient seine Position zwischen der Erfassung der Bilddaten des Bildes mit dem C-Bogen-Röntgengerät und der Erfassung der elektroanatomischen Mapping-Daten, so kann basierend auf der registrierten und ermittelten Positionsänderung des Patienten die zuvor ermittelte Koordinatentransformation entsprechend angepasst werden, so dass die elektroanatomischen Mapping-Daten und die Bilddaten des zuvor erzeugten Bildes weiterhin positions- und lagegenau miteinander fusioniert oder einander überlagert werden können.

Nach einer weiteren Variante der Erfindung wird wenigstens ein Teil eines mit dem elektromagnetischen Positionserfassungs- und Mappingsystems erfassbaren Katheters oder eines mit dem elektromagnetischen Positionserfassungs- und Mappingsystems erfassbaren Instrumentes unter Berücksichtigung der Position des Patienten in das Bild des Patienten und/oder die elektroanatomischen Mapping-Daten eingeblendet. Auch in diesem Fall erfolgt unter Berücksichtigung der Positionsänderung des Patienten eine Anpassung der Koordinatentransformation, so dass ein Abbild des Katheters oder eines anderen Instrumentes lage- und positionsgenau in ein aufgenommenes Bild von dem Patienten eingeblendet werden kann, das auch elektroanatomische Mapping-Daten aufweisen kann, die zuvor gewonnen wurden oder gleichzeitig während der Navigation des Katheters oder des Instrumentes erfasst werden.

Nach einer Ausführungsform der Erfindung wird der Patient zur Erfassung der Position des Patienten mit wenigstens einem Referenzsensor des elektromagnetischen Positionserfassungs- und Mappingsystems versehen, so dass die Position des Patienten während der Erfassung eines Bildes mit dem C-Bogen-Röntgengerät, während der Erfassung der elektroanatomischen Mapping-Daten und während der Katheteranwendung mit dem elektromagnetischen Positionserfassungs- und Mappingsystem in dem dem elektromagnetischen Positionserfassungs- und Mappingsystem zugeordneten Koordinatensystem ermittelt werden kann.

Nach einer Variante der Erfindung werden die mit dem elektromagnetischen Positionserfassungs- und Mappingsystem erfassten Positionen oder Koordinaten relativ zu dem Referenzsensor des Patienten erfasst. In diesem Fall ergibt sich eine direkte Registrierung zwischen dem einem Bild zugeordneten Koordinatensystem und einem über den Referenzsensor dem Patienten zugeordneten Koordinatensystem.

Nach einer Ausführungsform der Erfindung erfolgt eine zeitliche Synchronisation zwischen der Erfassung eines Bildes des Patienten mit dem C-Bogen-Röntgengerät und der Position des Patienten, wobei einem aufgenommenen Bild die Erfassungszeit zugeordnet wird und die Position des Patienten über der Zeit erfasst wird, so dass durch Zeitvergleich die Position des Patienten zur Erfassungszeit des Bildes ermittelbar ist und das erfasste Bild dieser Position des Patienten zugeordnet werden kann.

Nach einer anderen Ausführungsform der Erfindung ordnet das C-Bogen-Röntgengerät einem aufgenommenen Bild des Patienten zum Zeitpunkt der Erfassung des Bildes eine Identifikationskennung zu und übermittelt die Identifikationskennung zum Zeitpunkt der Erfassung des Bildes an das elektromagnetische Positionserfassungs- und Mappingsystem, wobei das elektromagnetische Positionserfassungs- und Mappingsystem die Position des Patienten inklusive der Identifikationskennung zum Zeitpunkt der Erfassung des Bildes speichert.

Nach einer weiteren Variante der Erfindung ruft das C-Bogen-Röntgengerät zum Zeitpunkt der Erfassung eines Bildes des Patienten von dem elektromagnetischen Positionserfassungs- und Mappingsystem die Position des Patienten ab und ordnet diese dem erfassten Bild zu.

Diese Varianten der Erfindung ermöglichen es jeweils, eine Positionsänderung des Patienten im Vergleich zu einer früher eingenommenen Position des Patienten, bei der ein Bild aufgenommen wurde, zu registrieren und die Positionsänderung bei der Überlagerung oder Fusion von Bilddaten und Mapping-Daten oder bei der Einblendung eines Katheters oder eines Instrumentes in die zuvor aufgenommenen Bilddaten zu berücksichtigen.

Ein Ausführungsbeispiel der Erfindung ist in den beigefügten schematischen Zeichnungen dargestellt. Es zeigen:
- FIG 1: eine Vorrichtung mit einem elektromagnetischen Positionserfassungs- und Mappingsystem, einem C-Bogen-Röntgengerät, Recheneinrichtungen sowie mit einer Patientenlagerungsvorrichtung und
- FIG 2: die Vorrichtung aus FIG 1 mit einem auf der Patientenlagerungsvorrichtung angeordneten Phantom.

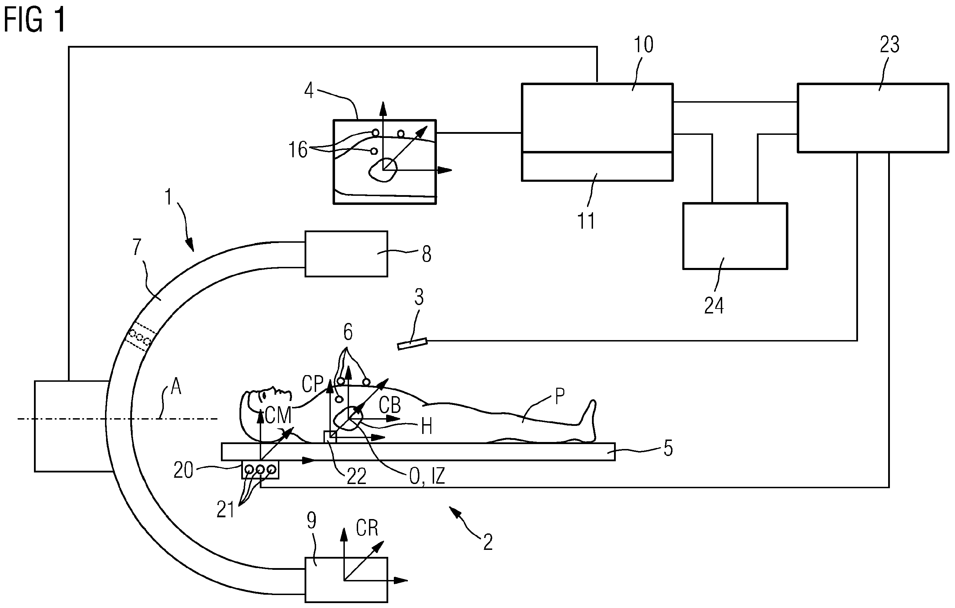

In FIG 1 ist eine Vorrichtung mit einem C-Bogen-Röntgengerät 1 und einem elektromagnetischen Positionserfassungs- und Mappingsystem 2 gezeigt, die zur visuellen Unterstützung einer Katheteranwendung an einem Lebewesen, beispielsweise einer Katheterablation am Herzen H des Patienten P dient. Im Falle des vorliegenden Ausführungsbeispiels soll ein Katheter 3 des elektromagnetischen Positionserfassungs- und Mappingsystems 2, der vorliegend sowohl ein Mapping-Katheter als auch ein Ablationskatheter ist, unterstützt durch wenigstens ein mit dem C-Bogen-Röntgengerät 1 vom Herzen H des Patienten P gewonnenes Bild und unterstützt durch mit dem elektromagnetischen Positionserfassungs- und Mappingsystem 2 gewonnene Mapping-Daten vom Herzen H des Patienten P durch Venen oder Arterien in die Herzkammer des Herzens H des Patienten P geführt werden, um dort zur Behandelung von Herzrhythmusstörungen des Patienten P eine Ablationsprozedur durchführen zu können. Hierzu sollen Bilddaten eines mit dem C-Bogen-Röntgengerät 1 gewonnenen Bildes und mit dem elektromagnetischen Positionserfassungs- und Mappingsystem 2 gewonnene elektroanatomische Mapping-Daten miteinander fusioniert oder einander überlagert werden, um den Eingriff an dem Patienten P zu erleichtern. Zudem soll die Position des in den Patienten P eingeführten Katheters 3 in die auf einem Sichtgerät 4 dargestellten miteinander fusionierten oder einander überlagerten Bilddaten und Mapping-Daten eingeblendet werden. Um die Bilddaten des mit dem C-Bogen-Röntgengerät 1 gewonnenen Bildes und die mit dem elektromagnetischen Positionserfassungs- und Mappingsystem gewonnenen elektroanatomischen Mapping-Daten miteinander fusionieren oder einander überlagern zu können bzw. um ein Abbild des Katheters 3 in die fusionierten oder überlagerten Daten lage- und positionsgenau einblenden zu können, werden das C-Bogen-Röntgengerät 1 und das elektromagnetische Positionserfassungs- und Mappingsystem 2 relativ zueinander kalibriert.

Hierzu wird der Patient P auf einer dem C-Bogen-Röntgengerät 1 definiert zugeordneten Patientenlagerungsvorrichtung 5 gelagert. Unter einer definierten Zuordnung von C-Bogen-Röntgengerät 1 und Patientenlagerungsvorrichtung 5 ist dabei zu verstehen, dass die räumliche Anordnung der beiden Gerätschaften auch bei einer Verstellung der Gerätschaften oder von Teilen der Gerätschaften relativ zueinander bekannt ist. Der Patient P wird im Bereich seines Herzens H vorzugsweise mit wenigstens drei röntgenpositiven Marken 6 versehen. Die Marken 6 werden so an dem Patienten P angeordnet, dass diese in mit dem C-Bogen-Röntgengerät 1 aufnehmbaren Röntgenbildern vom Herzen H des Patienten P abgebildet werden, ohne Details vom Herzen H zu verdecken, und dass diese mit dem elektromagnetischen Positionserfassungs- und Mappingsystem 2 detektiert werden können. Auf diese Weise kann anhand der Marken 6 eine Koordinatentransformation zwischen einem dem C-Bogen-Röntgengerät 1 zugeordneten Koordinatensystem CR bzw. zwischen einem mit dem C-Bogen-Röntgengerät 1 erzeugten Bild zugeordneten Koordinatensystem CB und einem dem elektromagnetischen Positionserfassungs- und Mappingsystem 2 zugeordneten Koordinatensystem CM ermittelt werden.

Im Falle des vorliegenden Ausführungsbeispiels wird mit dem C-Bogen-Röntgengerät 1 ein Volumendatensatz bzw. ein 3D-Bild vom Herzen H des Patienten P gewonnen. Der mit einer Röntgenstrahlenquelle 8 und einem Röntgenstrahlenempfänger 9 versehene C-Bogen 7 des C-Bogen-Röntgengerätes 1 wird dabei um seine Orbitalachse O oder seine Angulationsachse A in einem Winkelbereich von ca. 190° verschwenkt, wobei eine Vielzahl von 2D-Röntgenprojektionen vom Herzen H des Patienten P aus unterschiedlichen Projektionsrichtungen aufgenommen werden, aus denen ein Bildrechner 10 in an sich bekannter Weise einen Volumendatensatz bzw. ein 3D-Bild vom Herzen H des Patienten P rekonstruiert. Da es sich bei dem Herzen H um ein bewegtes Organ handelt, erfolgt die Rekonstruktion des Volumendatensatzes bzw. des 3D-Bildes vom Herzen H im Falle des vorliegenden Ausführungsbeispiels unter Verwendung von von dem Patienten P während der Gewinnung der 2D-Röntgenprojektionen aufgezeichneten EKG-Signalen. Das EKG-Equipment ist in FIG 1 nicht dargestellt, da es in an sich bekannter Weise ausgeführt ist. Ein derartiges, EKG-getriggertes Rekonstruktionsverfahren ist beispielsweise in der DE 10 2005 016 472 A1 beschrieben.

In dem rekonstruierten 3D-Bild vom Herzen H des Patienten P sind auch die Marken 6 abgebildet. Zur Vorbereitung der Berechnung der zuvor erwähnten Koordinatentransformation werden die Abbilder 16 der Marken 6 manuell oder auch automatisch mit einem Verfahren der Mustererkennung in dem 3D-Bild lokalisiert und die Koordinaten der Marken 6 in dem dem 3D-Bild zugeordneten Koordinatensystem CB bzw. dem dem C-Bogen-Röntgengerät 1 zugeordnete Koordinatensystem CR ermittelt, was möglich ist da die Abmessungen und Geometrien des C-Bogen-Röntgengerätes 1 sowie die Projektionsgeometrien für die Gewinnung der 2D-Röntgenprojektionen bekannt sind. Der Koordinatenursprung des Koordinatensystem CB kann dabei beispielsweise im Isozentrum IZ des C-Bogens 7 lokalisiert werden. Wie bereits erwähnt, sind die Marken 6 derart am Patienten P angeordnet, dass diese keine interessierenden Strukturen des Herzens H verdecken. Der Volumendatensatz bzw. das 3D-Bild vom Herzen H des Patienten P wird schließlich in einem Bildspeicher 11 zwischengespeichert, um die 3D-Bilddaten für eine Fusion oder Überlagerung mit anderen Daten bzw. für eine Navigation des Katheters 3 oder eines anderen Instrumentes verwenden zu können.

Das elektromagnetische Positionserfassungs- und Mappingsystem 2 weist im Falle des vorliegenden Ausführungsbeispiels drei in einer Einheit 20 zusammengefasste Transmitter 21 auf, die jeweils ein elektromagnetisches Wechselfeld erzeugen. Die drei Wechselfelder unterscheiden sich dabei voneinander. Die Einheit 20 mit den drei Transmittern 21 ist im Falle des vorliegenden Ausführungsbeispiels abnehmbar an einer definierten Stelle der Patientenlagerungsvorrichtung 5 angeordnet. Sollte die Einheit 20 beispielsweise bei der Aufnahme der 2D-Röntgenprojektionen stören kann sie demnach von der Patientenlagerungsvorrichtung 5 abgenommen und nach der Aufnahme der 2D-Röntgenprojektionen wieder an der Patientenlagerungsvorrichtung 5 angeordnet werden. Die Einheit 20 mit den drei Transmittern 21 könnte auch an einer anderen Stelle der Patientenlagerungsvorrichtung 5 oder des definiert zu der Patientenlagerungsvorrichtung 5 angeordneten C-Bogen-Röntgengerätes 1 definiert abnehmbar angeordnet sein, wie dies beispielsweise in FIG 1 am C-Bogen 7 des C-Bogen-Röntgengerätes 1 mit gestrichelten Linien angedeutet ist.

Der Katheter 3 des elektromagnetischen Positionserfassungs-und Mappingsystems 2 ist in in FIG 1 nicht näher dargestellter Weise in seiner Katheterspitze mit drei Sensoren versehen. Wird der Katheter 3 in den Wechselfeldern der Transmitter 21 bewegt, kann die Position des Katheters 3 mit einer Recheneinrichtung 23 des elektromagnetischen Positionserfassungs- und Mappingsystem 2 in dem dem elektromagnetischen Positionserfassungs- und Mappingsystem 2 zugeordneten Koordinatensystem CM ermittelt werden.

Im Falle des vorliegenden Ausführungsbeispiels ist der Patient P an seinem Rücken mit einem Referenzsensor 22 des elektromagnetischen Positionserfassungs- und Mappingsystems 2 versehen, so dass auch Bewegungen des Patienten P mit dem elektromagnetischen Positionserfassungs- und Mappingsystem ermittelt werden können. Dem Referenzsensor 22 bzw. dem Patienten P ist ein Patientenkoordinatensystem CP zugeordnet.

Für die Ermittlung der Koordinatentransformation zwischen dem dem C-Bogen-Röntgengerät 1 zugeordneten Koordinatensystem CR bzw. dem mit dem C-Bogen-Röntgengerät 1 gewonnenen 3D-Bild zugeordneten Koordinatensystem CB und dem dem elektromagnetischen Positionserfassungs- und Mappingsystem 2 zugeordneten Koordinatensystem CM bzw. dem Patientenkoordinatensystem CP werden die Koordinaten der Marken 6 in dem dem elektromagnetischen Positionserfassungs- und Mappingsystem 2 zugeordneten Koordinatensystem CM bzw. dem Patientenkoordinatensystem CP ermittelt, indem die Marken 6 mit dem Katheter 3 berührt werden. Bevorzugt, aber nicht notwendigerweise werden die Koordinaten der Marken 6 im Patientenkoordinatensystem CP angegeben.

Liegen die Koordinaten der Marken 6 in dem dem Volumendatensatz bzw. dem 3D-Bild zugeordneten Koordinatensystem CB im Bildrechner 10 sowie die Koordinaten der Marken 6 in dem Patientenkoordinatensystem CP bzw. in dem dem elektromagnetischen Positionserfassungs- und Mappingsystem 2 zugeordneten Koordinatensystem CM in der Recheneinrichtung 23 des elektromagnetischen Positionserfassungs- und Mappingsystems 2 vor, können mit dem Bildrechner 10, der Recheneinrichtung 23 oder einem weiteren Rechner 24 die jeweilige Koordinatentransformationen ermittelt werden.

Vor oder während einer Katheteranwendung am Herzen H des Patienten P können mit dem Katheter 3 des Weiteren elektroanatomische Mapping-Daten, vorzugsweise 3D-Mapping-Daten, gewonnen werden. Mittels der in die Katheterspitze des Katheters 3 integrierten elektromagnetischen Sensoren ist es möglich, die durch Katheterbewegungen des Katheters 3 induzierten Spannungsänderungen innerhalb der Wechselfelder der Transmitter zu messen und mit Hilfe mathematischer Algorithmen zu jedem Zeitpunkt die Position des Katheters 3 zu messen. Durch punktweises Abtasten von Bereichen einer Herzkammer mit dem Katheter 3 bei simultaner Erfassung der elektrischen Signale der Sensoren entsteht somit eine elektroanatomisch dreidimensionale Landkarte bzw. entstehen 3D-Mapping-Daten, wobei die elektrischen Signale z. B. farbkodiert wiedergegeben werden können. Da die Koordinatentransformation zwischen dem dem mit dem C-Bogen-Röntgengerät 1 erzeugten 3D-Bild zugeordneten Koordinatensystem CB und dem Patientenkoordinatensystem CP bzw. dem dem elektromagnetischen Positionserfassungs- und Mappingsystem 2 zugeordneten Koordinatensystem CM bekannt ist, können nunmehr die Bilddaten des 3D-Bildes und die 3D-Mapping-Daten zur visuellen Unterstützung einer Katheteranwendung am Herzen H des Patienten P miteinander fusioniert bzw. einander überlagert und auf dem Sichtgerät 4 dargestellt werden. Zusätzlich kann der Katheter 3 beispielsweise anhand der fusionierten Daten relativ zum Herzen H des Patienten P navigiert werden, indem ein Abbild des Katheters 3 basierend auf der bekannten Koordinatentransformation in entsprechender Weise in die miteinander fusionierten oder einander überlagerten Daten eingeblendet wird.

Es wird also deutlich, dass durch das erfindungsgemäße Vorgehen vor einer Katheteranwendung mit einem Bildgebungsgerät erzeugte Bilddaten mit ebenfalls vor der Katheteranwendung oder, nicht erfindungsgemäß, während der Katheteranwendung gewonnen 3D-Mapping-Daten fusioniert oder überlagert werden können und ein Katheter 3 anhand der fusionierten oder überlagerten Daten relativ zu einem Patienten P navigiert werden kann.

Um zu vermeiden, dass nach einer Positionsänderung des Patienten P nach der Erfassung des 3D-Bildes, wonach die ermittelte Koordinatentransformation für das 3D-Bild nicht mehr gültig wäre, die Koordinatentransformation neu ermittelt werden muss, ist der Patient P, wie bereits erwähnt, mit einem Referenzsensor 22 des elektromagnetischen Positionserfassungs- und Mappingsystems 2 versehen, so dass durch Detektion des Referenzsensors 22 Positionsänderungen des Patienten P erfasst und die Koordinatentransformation beispielsweise der Bewegung des Patienten P entsprechend modifiziert werden kann.

Um eine Positionsänderung des Patienten P richtig berücksichtigen zu können, erfolgt nach einer Betriebsart eine zeitliche Synchronisation zwischen der Erfassung des 3D-Bildes mit dem C-Bogen-Röntgengerät 1 und der Position des Patienten P, wobei dem aufgenommenen 3D-Bild die Erfassungszeit t1 des 3D-Bildes zugeordnet wird und die Position des Patienten P mit dem Referenzsensor 22 über der Zeit, beispielsweise jede Sekunde, erfasst wird, so dass durch einen manuell oder auch automatisch vorgenommenen Zeitvergleich die Position des Patienten P zur Erfassungszeit t1 des 3D-Bildes ermittelbar ist. Auf diese Weise ist also eine Positionsänderung des Patienten P nach der Erfassung des 3D-Bildes ermittelbar und bei Transformationsberechnungen, insbesondere bei der Fusion oder Überlagerung von Bilddaten des 3D-Bildes und aktuell aufgenommenen Mapping-Daten oder bei der aktuellen Navigation des Katheters 3 basierend auf dem 3D-Bild berücksichtigbar. Für die zeitliche Synchronisation weist vorzugsweise sowohl der Bildrechner 10 als auch die Recheneinrichtung 23 eine Uhr auf, die beispielsweise über den Rechner 24 miteinander synchronisiert sind.

Die Position des Patienten P bei der Erfassung des 3D-Bildes kann auch derart festgehalten werden, dass das C-Bogen-Röntgengerät 1 bzw. der Bildrechner 10 dem 3D-Bild zum Zeitpunkt der Erfassung des 3D-Bildes eine Identifikationskennung zuordnet und die Identifikationskennung zum Zeitpunkt der Erfassung des 3D-Bildes in Realtime an das elektromagnetischen Positionserfassungs- und Mappingsystem 2 bzw. die Recheneinrichtung 23 des elektromagnetischen Positionserfassungs- und Mappingsystems 2 übermittelt, wobei die Recheneinrichtung 23 des elektromagnetischen Positionserfassungs- und Mappingsystems 2 die Position des Patienten P inklusive der Identifikationskennung zum Zeitpunkt der Erfassung des 3D-Bildes speichert.

Des Weiteren kann das C-Bogen-Röntgengerät 1 bzw. der Bildrechner 10 zum Zeitpunkt der Erfassung des 3D-Bildes des Patienten P von der Recheneinrichtung 23 des elektromagnetischen Positionserfassungs- und Mappingsystems 2 die Position des Patienten P abrufen und dem erfassten 3D-Bild zuordnen.

In all diesen Fällen ist dem erfassten 3D-Bild des Patienten P die Position des Patienten P bei der Erfassung des 3D-Bildes zugeordnet, so dass selbst bei einer Positionsänderung des Patienten P gegenüber der Position, die der Patient P bei der Erfassung des 3D-Bildes eingenommen hat, die einmal ermittelte Transformationsbeziehung nicht neu ermittelt werden muss, sondern entsprechend der Positionsänderung des Patienten P entsprechend angepasst werden kann. Die Fusion oder Überlagerung aktuell ermittelter Mapping-Daten mit Bilddaten des vor der Positionsänderung ermittelten 3D-Bildes bzw. die Einblendung aktueller Abbilder des Katheters 3 in die Bilddaten des vor der Positionsänderung ermittelten 3D-Bildes erfolgt dann auf Basis der der neuen Position des Patienten P entsprechend modifizierten Transformationsbeziehung.

Sollte die Transmittereinheit 20 beispielsweise aus Platzgründen während der Gewinnung des Volumendatensatzes bzw. des 3D-Bildes mit dem C-Bogen-Röntgengerät von der Patientenlagerungsvorrichtung 5 entfernt werden müssen, so kann vor der Entfernung und nach der Wiederanordnung der Transmittereinheit 20 die Position des Patienten P ermittelt werden. Hat sich der Patient P während der Gewinnung des Volumendatensatzes bzw. des 3D-Bildes bewegt, so ist in Abhängigkeit von den ermittelten Positionswerten vom Anwender zu entscheiden, ob der vor der Entfernung der Transmittereinheit 20 aufgenommene Positionswert, der nach der Wiederanordnung der Transmittereinheit 20 aufgenommene Positionswert oder ein Durchschnittswert dem Volumendatensatz bzw. dem 3D-Bild als Positionswert zugeordnet wird. Auch bei diesem Vorgehen wird die Position des Patienten P während der Erfassung des Volumendatensatzes bzw. des 3D-Bildes ermittelt.

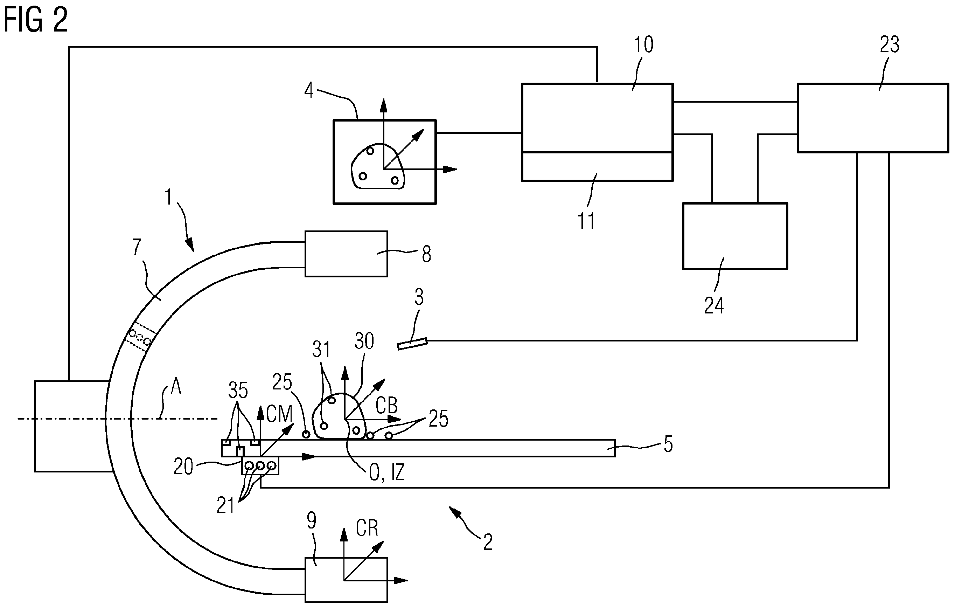

Anhand von FIG 1 wurde die Ermittlung der Koordinatentransformation zwischen dem dem C-Bogen-Röntgengerät 1 zugeordneten Koordinatensystem CR bzw. dem mit dem C-Bogen-Röntgengerät 1 gewonnenen 3D-Bild zugeordneten Koordinatensystem CB und dem dem elektromagnetischen Positionserfassungs- und Mappingsystem 2 zugeordneten Koordinatensystem CM bzw. dem Patientenkoordinatensystem CP beschrieben, wobei Marken 6 an dem Patienten P angeordnet wurden. Die Ermittlung der Koordinatentransformation kann jedoch auch in einem sogenannten Offline-Vorgang, d.h. ohne Patienten P, vorgenommen werden. Dies kann wie in FIG 2 dargestellt beispielsweise derart erfolgen, dass vorzugsweise drei röntgenpositive Positionssensoren 25 des elektromagnetischen Positionserfassungs-und Mappingsystems 2, beispielsweise drei Katheter 3, an der Patientenlagerungsvorrichtung 5 angeordnet und die Koordinaten der Positionssensoren 25 in dem dem elektromagnetischen Positionserfassungs- und Mappingsystem 2 zugeordneten Koordinatensystem CM ermittelt werden. Außerdem werden mit dem C-Bogen-Röntgengerät 1 wenigstens zwei Röntgenprojektionsbilder unter voneinander verschiedenen Projektionsrichtungen oder ein Röntgen-3D-Bild von den Positionssensoren 25 gewonnen, die Abbilder der Positionssensoren 25 manuell oder mit einem Verfahren der Mustererkennung in den Bildern bzw. dem 3D-Bild lokalisiert und die Koordinaten der Positionssensoren in dem dem C-Bogen-Röntgengerät 1 zugeordneten Koordinatensystem CR bzw. in dem den Projektionsbildern bzw. in dem dem 3D-Bild zugeordneten Koordinatensystem CB ermittelt. Wird basierend auf den in den Koordinatensystemen ermittelten Koordinaten die Koordinatentransformation ermittelt sind das C-Bogen-Röntgengerät 1 und das elektromagnetische Positionserfassungs- und Mappingsystem 2 relativ zueinander kalibriert.

Alternativ kann ein Phantom 30 verwendet werden, das mit röntgenpositiven Marken 31 versehen ist. Wird mit dem C-Bogen-Röntgengerät 1 beispielsweise ein 3D-Bild von dem Phantom 30 erzeugt, indem die röntgenpositiven Marken 31 abgebildet sind, so können die Abbilder der Marken 31 wieder manuell durch händisches Anklicken oder automatisch mit einem Verfahren der Mustererkennung in dem 3D-Bild lokalisiert und basierend darauf aufgrund der bekannten Konstruktion des C-Bogen-Röntgengerätes 1 und der bekannten Projektionsgeometrien der dem 3D-Bild zugrundeliegenden 2D-Projektionen die Koordinaten der Marken 31 in dem dem C-Bogen-Röntgengerät 1 zugeordneten Koordinatensystem CR bzw. in dem dem 3D-Bild zugeordneten Koordinatensystem CB ermittelt werden. Die Ermittlung der Koordinaten der Marken 31 in dem dem elektromagnetischen Positionserfassungs- und Mappingsystem 2 zugeordneten Koordinatensystem CM erfolgt beispielsweise durch Berührung der Marken 31 mit dem Katheter 3. Anschließend kann wieder die Koordinatentransformation anhand der in den Koordinatensystemen ermittelten Koordinaten berechnet werden.

Alternativ können alle Marken 31 des Phantoms 30 Positionssensoren des elektromagnetischen Positionserfassungs- und Mappingsystems 2 sein, so dass zur Ermittlung der Koordinaten der Marken 31 in dem Koordinatensystem CM des elektromagnetischen Positionserfassungs- und Mappingsystems 2 die Marken 31 nicht extra mit dem Katheter 3 berührt werden müssen.

Alternativ kann auch nur eine Marke 31 des Phantoms 30 ein Positionssensor des elektromagnetischen Positionserfassungs-und Mappingsystems 2 sein. Sind die Positionen der übrigen Marken 31 des Phantoms 30 dabei relativ zu dem wenigstens einen Positionssensor des Phantoms 30 bekannt, so müssen nur die Koordinaten dieses einen Positionssensors in dem Koordinatensystem CM des elektromagnetischen Positionserfassungs-und Mappingsystems 2 ermittelt werden, während sich die anderen aufgrund ihrer bekannten Positionen zu dem detektierten Positionssensor des Phantoms 30 ermitteln lassen.

Eine weitere Möglichkeit, eine Beziehung zwischen dem dem C-Bogen-Röntgengerät 1 zugeordneten Koordinatensystem CR und dem dem elektromagnetischen Positionserfassungs- und Mappingsystem 2 zugeordneten Koordinatensystem CM herzustellen, besteht darin, beispielsweise drei Positions- und Orientierungssensoren 35 des elektromagnetischen Positionserfassungs-und Mappingsystems 2 an oder in der Patientenlagerungsvorrichtung 5 definiert anzuordnen. In diesem Fall ist aufgrund der bekannten konstruktiven Beziehung zwischen dem C-Bogen-Röntgengerät 1 und der Patientenlagerungsvorrichtung 5 und der bekannten konstruktiven Anordnung der drei Positions- und Orientierungssensoren 35 an oder in der Patientenlagerungsvorrichtung 5 die Transformationsbeziehung zwischen dem dem C-Bogen-Röntgengerät 1 zugeordneten Koordinatensystem CR und damit auch zwischen dem einem mit dem C-Bogen-Röntgengerät 1 aufgenommenen Bild zugeordneten Koordinatensystem CB und dem dem elektromagnetischen Positionserfassungs- und Mappingsystem 2 zugeordneten Koordinatensystem CM durch Erfassung der drei Positions- und Orientierungssensoren 35 mit dem elektromagnetischen Positionserfassungs- und Mappingsystem 2 ermittelbar. Die drei Positions- und Orientierungssensoren 35 des elektromagnetischen Positionserfassungs- und Mappingsystems 2 können dabei von der Patientenlagerungsvorrichtung abnehmbar ausgeführt sein, in dem diese beispielsweise in einem oder mehreren abnehmbaren Modulen angeordnet sind.

Das C-Bogen-Röntgengerät 1 und das elektromagnetischen Positionserfassungs- und Mappingsystem 2 können demnach auch offline relativ zueinander kalibriert werden. Im Anschluss daran können dann Patientenaufnahmen mit dem C-Bogen-Röntgengerät 1 und dem elektromagnetischen Positionserfassungs- und Mappingsystem 2 erfolgen und mit dem C-Bogen-Röntgengerät gewonnene Bilddaten und Mapping-Daten des elektromagnetischen Positionserfassungs- und Mappingsystems 2 miteinander fusioniert oder einander überlagert werden. Des Weiteren sind Abbilder von mit dem elektromagnetischen Positionserfassungs- und Mappingsystem 2 erfassbaren Navigationsinstrumenten wie dem Katheter 3 in die mit dem C-Bogen-Röntgengerät 1 erfassten Bilder und/oder in die Mapping-Daten und/oder in miteinander fusionierte oder einander überlagerte Daten einblendbar. Positionsänderungen des Patienten P können dabei, wie bereits beschrieben, durch Detektion des Referenzsensors 22 registriert, ermittelt und entsprechend berücksichtigt werden.

Die an dem Patienten angeordneten Marken können im Übrigen auch Positions- und Orientierungssensoren des elektromagnetischen Positionserfassungs- und Mappingsystems sein.

Des Weiteren können an dem Patienten P mehrere Referenzsensoren angeordnet sein.

Wie bereits erwähnt können außer dem Katheter 3 auch andere mit einem Positions- und Orientierungssensor versehene Instrumente bei der beschriebenen Vorrichtung eingesetzt werden. Wird z.B. anstelle oder zusätzlich zu dem Katheter 3 ein Katheter eingesetzt mit dem Bildinformationen aus dem Körperinneren gewonnene werden können, beispielsweise dem Ultraschall-Katheter AcuNav von Siemens Medical Solutions, so können die mit diesem Katheter gewonnenen Bilddaten mit Bilddaten des mit dem Bildgebungsgerät gewonnenen 3D-Bildes kombiniert werden. Auch Katheter-Steuerungssysteme wie das Niobe System von Stereotaxis kann bei der Vorrichtung eingesetzt werden.

## Patentansprüche

1. Verfahren zur visuellen Unterstützung einer Katheteranwendung am Herzen (H) eines Patienten (P) anhand von wenigstens einem mit einem C-Bogen-Röntgengerät (1) von dem Patienten (P) gewonnenen Bild und anhand von mit einem elektromagnetischen Positionserfassungs- und Mappingsystem (2) gewonnenen elektroanatomischen Mapping-Daten von dem Patienten (P), bei dem das wenigstens eine Bild von dem Patienten und die elektroanatomischen Mapping-Daten von dem Patienten miteinander fusioniert oder einander überlagert werden und bei dem das C-Bogen-Röntgengerät (1) und das elektromagnetische Positionserfassungs- und Mappingsystem (2) relativ zueinander kalibriert werden, indem eine Koordinatentransformation zwischen einem dem C-Bogen-Röntgengerät (1) zugeordneten Koordinatensystem (CR) und/oder einem wenigstens einem mit dem C-Bogen-Röntgengerät (1) erzeugten Bild zugeordneten Koordinatensystem (CB) und einem dem elektromagnetischen Positionserfassungs- und Mappingsystem (2) zugeordneten Koordinatensystem (CM) ermittelt wird, und bei dem die Position des Patienten (P) während der Erfassung des Bildes und während der Erfassung der elektroanatomischen Mapping-Daten, die vor der Katheteranwendung gewonnen wurden, ermittelt und dem Bild und den elektroanatomischen Mapping-Daten zumindest indirekt zugeordnet wird, wobei das C-Bogen-Röntgengerät (1) einem aufgenommenen Bild des Patienten (P) zum Zeitpunkt der Erfassung des Bildes eine Identifikationskennung zuordnet und die Identifikationskennung zum Zeitpunkt der Erfassung des Bildes an das elektromagnetische Positionserfassungs- und Mappingsystem (2) übermittelt, wobei das elektromagnetische Positionserfassungs- und Mappingsystem (2) die Position des Patienten (P) inklusive der Identifikationskennung zum Zeitpunkt der Erfassung des Bildes speichert.

2. Verfahren, insbesondere nach Anspruch 1, zur visuellen Unterstützung einer Katheteranwendung am Herzen (H) eines Patienten (P) anhand von wenigstens einem mit einem C-Bogen-Röntgehgerät (1) von dem Patienten (P) gewonnenen Bild und anhand von mit einem elektromagnetischen Positionserfassungs- und Mappingsystem (2) gewonnenen elektroanatomischen Mapping-Daten von dem Patienten (P), bei dem das wenigstens eine Bild von dem Patienten und die elektroanatomischen Mapping-Daten von dem Patienten miteinander fusioniert oder einander überlagert werden und bei dem das C-Bogen-Röntgengerät (1) und das elektromagnetische Positionserfassungs- und Mappingsystem (2) relativ zueinander kalibriert werden, indem eine Koordinatentransformation zwischen einem dem C-Bogen-Röntgengerät (1) zugeordneten Koordinatensystem (CR) und/oder einem wenigstens einem mit dem C-Bogen-Röntgengerät (1) erzeugten Bild zugeordneten Koordinatensystem (CB) und einem dem elektromagnetischen Positionserfassungs- und Mappingsystem (2) zugeordneten Koordinatensystem (CM) ermittelt wird, und bei dem die Position des Patienten (P) während der Erfassung des Bildes und während der Erfassung der elektroanatomischen Mapping-Daten, die vor der Katheteranwendung gewonnen wurden, ermittelt und dem Bild und den elektroanatomischen Mapping-Daten zumindest indirekt zugeordnet wird, wobei eine zeitliche Synchronisation zwischen der Erfassung eines Bildes des Patienten (P) mit dem C-Bogen-Röntgengerät (1) und der Position des Patienten (P) erfolgt, wobei einem aufgenommenen Bild die Erfassungszeit zugeordnet wird und die Position des Patienten (P) über der Zeit erfasst wird, so dass durch Zeitvergleich die Position des Patienten (P) zur Erfassungszeit des Bildes ermittelbar ist.

3. Verfahren nach Anspruch 1 oder 2, bei dem die Ermittlung der Koordinatentransformation zwischen dem dem C-Bogen-Röntgengerät (1) zugeordneten Koordinatensystem (CR) und/oder dem dem wenigstens einen mit dem C-Bogen-Röntgengerät (1) erzeugten Bild zugeordneten Koordinatensystem (CB) und dem dem elektromagnetischen Positionserfassungs-und Mappingsystem (2) zugeordneten Koordinatensystem (CM) anhand von wenigstens einer Marke (6, 25, 31) erfolgt, welche in einem erzeugten Bild abgebildet ist und mit dem elektromagnetischen Positionserfassungs- und Mappingsystem (2) detektierbar ist.

4. Verfahren nach Anspruch 3, bei dem die Marke (6,25, 31) eine röntgenpositive Marke (6, 25, 31) ist und in wenigstens zwei unter voneinander verschiedenen Projektionswinkeln aufgenommenen Röntgenprojektionen oder in einem 3D-Bild abgebildet ist.

5. Verfahren nach Anspruch 4, bei dem das Abbild (16) der Marke (6) in den Projektionsbildern oder dem 3D-Bild manuell oder automatisch mit einem Verfahren der Mustererkennung lokalisiert wird.

6. Verfahren nach einem der Ansprüche 3 bis 5, bei dem die Marke (6, 31) mit einem Positionssensor (3) des elektromagnetischen Positionserfassungs- und Mappingsystems (2) berührt wird.

7. Verfahren nach einem der Ansprüche 3 bis 6, bei dem wenigstens eine Marke ein röntgenpositiver Positionssensor (25) des elektromagnetischen Positionserfassungs- und Mappingsystems (2) ist.

8. Verfahren nach einem der Ansprüche 3 bis 7, bei dem ein mit wenigstens einer Marke (31) versehenes Phantom (30) vorgesehen ist.

9. Verfahren nach Anspruch 8, bei dem wenigstens eine Marke des Phantoms (30) ein Positionssensor des elektromagnetischen Positionserfassungs- und Mappingsystems (2) ist.

10. Verfahren nach Anspruch 9, bei dem alle Marken des Phantoms (30) Positionssensoren des elektromagnetischen Positionserfassungs- und Mappingsystems (2) sind.

11. Verfahren nach Anspruch 9, bei dem das Phantom (30) neben dem Positionssensor des elektromagnetischen Positionserfassungs- und Mappingsystems (2) wenigstens eine weitere Marke aufweist, wobei die Position der wenigstens einen weiteren Marke des Phantoms (30) relativ zu dem wenigstens einen Positionssensor des Phantoms (30) bekannt ist.

12. Verfahren nach einem der Ansprüche 8 bis 11, bei dem das Phantom (30) und das elektromagnetische Positionserfassungs- und Mappingsystem (2) derart definiert relativ zueinander angeordnet werden, dass die Koordinatentransformation zwischen einem dem Phantom (30) zugeordneten Koordinatensystem und einem dem elektromagnetischen Positionserfassungs-und Mappingsystem (2) zugeordneten Koordinatensystem (CM) bekannt ist.

13. Verfahren nach einem der Ansprüche 1 bis 12, bei dem das C-Bogen-Röntgengerät (1) zum Zeitpunkt der Erfassung eines Bildes des Patienten (P) von dem elektromagnetischen Positionserfassungs- und Mappingsystem (2) die Position des Patienten (2) abruft und dem erfassten Bild zuordnet.

14. Verfahren nach einem der Ansprüche 1 bis 13, bei dem bei einer Fusion oder Überlagerung von vor einer Katheteranwendung mit dem C-Bogen-Röntgengerät (1) aufgenommenen Bilddaten eines Bildes des Patienten (P) mit vor oder während einer Katheteranwendung aufgenommenen elektroanatomischen Mapping-Daten des Patienten (P) oder bei einer Navigation eines Katheters (3) basierend auf vor einer Katheteranwendung mit dem C-Bogen-Röntgengerät (1) aufgenommenen Bilddaten eines Bildes des Patienten (P) und/oder basierend auf vor einer Katheteranwendung aufgenommenen elektroanatomischen Mapping-Daten eine Veränderung der Position des Patienten (P) in Bezug auf die aufgenommenen Bilddaten berücksichtigt wird.

15. Vorrichtung zur visuellen Unterstützung einer Katheteranwendung am Herzen (H) eines Patienten (P) anhand von wenigstens einem mit einem C-Bogen-Röntgengerät (1) von dem Patienten (P) gewonnenen Bild und anhand von mit einem elektromagnetischen Positionserfassungs- und Mappingsystem (2) gewonnenen elektroanatomischen Mapping-Daten von dem Patienten (P), wobei das wenigstens eine Bild von dem Patienten und die elektroanatomisehen Mapping-Daten von dem Patienten miteinander fusioniert oder einander überlagert werden, aufweisend ein C-Bogen-Röntgengerät (1), ein elektromagnetisches Positionserfassungs-und Mappingsystem (2) und wenigstens eine Recheneinrichtung (10, 23, 24), die zur Ermittlung einer Koordinatentransformation zwischen einem dem C-Bogen-Röntgengerät (1) zugeordneten Koordinatensystem (CR) und/oder einem wenigstens einem mit dem C-Bogen-Röntgengerät (1) erzeugten Bild zugeordneten Koordinatensystem (CB) und einem dem elektromagnetischen Positionserfassungs- und Mappingsystem (2) zugeordneten Koordinatensystem (CM) eingerichtet ist, wobei die Position des Patienten (P) während der Erfassung des Bildes und während der Erfassung der elektroanatomischen Mapping-Daten, die vor der Katheteranwendung gewonnen wurden, ermittelt und dem Bild und den elektroanatomischen Mapping-Daten zumindest indirekt zugeordnet wird, wobei das C-Bogen-Röntgengerät (1) einem aufgenommenen Bild des Patienten (P) zum Zeitpunkt der Erfassung des Bildes eine Identifikationskennung zuordnet und die Identifikationskennung zum Zeitpunkt der Erfassung des Bildes an das elektromagnetische Positionserfassungs- und Mappingsystem (2) übermittelt, wobei das elektromagnetische Positionserfassungs- und Mappingsystem (2) die Position des Patienten (P) inklusive der Identifikationskennung zum Zeitpunkt der Erfassung des Bildes speichert.

16. Vorrichtung, insbesondere nach Anspruch 15, zur visuellen Unterstützung einer Katheteranwendung am Herzen (H) eines Patienten (P) anhand von wenigstens einem mit einem C-Bogen-Röntgengerät (1) von dem Patienten (P) gewonnenen Bild und anhand von mit einem elektromagnetischen Positionserfassungs- und Mappingsystem (2) gewonnenen elektroanatomischen Mapping-Daten von dem Patienten (P), wobei das wenigstens eine Bild von dem Patienten und die elektroanatomischen Mapping-Daten von dem Patienten miteinander fusioniert oder einander überlagert werden, aufweisend ein C-Bogen-Röntgengerät (1), ein elektromagnetisches Positionserfassungs-und Mappingsystem (2) und wenigstens eine Recheneinrichtung (10, 23, 24), die zur Ermittlung einer Koordinatentransformation zwischen einem dem C-Bogen-Röntgengerät (1) zugeordneten Koordinatensystem (CR) und/oder einem wenigstens einem mit dem C-Bogen-Röntgengerät (1) erzeugten Bild zugeordneten Koordinatensystem (CB) und einem dem elektromagnetischen Positionserfassungs- und Mappingsystem (2) zugeordneten Koordinatensystem (CM) eingerichtet ist, wobei die Position des Patienten (P) während der Erfassung des Bildes und während der Erfassung der elektroanatomischen Mapping-Daten, die vor der Katheteranwendung gewonnen wurden, ermittelt und dem Bild und den elektroanatomischen Mapping-Daten zumindest indirekt zugeordnet wird, wobei eine zeitliche Synchronisation zwischen der Erfassung eines Bildes des Patienten (P) mit dem C-Bogen-Röntgengerät (1) und der Position des Patienten (2) erfolgt, wobei einem aufgenommenen Bild die Erfassungszeit zugeordnet wird und die Position des Patienten (P) über der Zeit erfasst wird, so dass durch Zeitvergleich die Position des Patienten (P) zur Erfassungszeit des Bildes ermittelbar ist.

17. Vorrichtung nach Anspruch 15 oder 16, bei der dem C-Bogen-Röntgengerät (1) eine Patientenlagerungsvornchtung (5) definiert zugeordnet ist.

18. Vorrichtung nach einem der Ansprüche 15 bis 17, bei der das elektromagnetische Positionserfassungs- und Mappingsystem (2) wenigstens einen Transmitter (21) zur Erzeugung eines elektromagnetischen Wechselfeldes und einen Katheter (3) mit wenigstens einem Sensor aufweist.

19. Vorrichtung nach Anspruch 18, bei der der Transmitter (21) an dem C-Bogen-Röntgengerät (1) oder an der Patientenlagerungsvornchtung (5) definiert angeordnet ist.

20. Vorrichtung nach Anspruch 18 oder 19, bei der der Transmitter (21) an dem C-Bogen (7) des C-Bogen-Röntgengerätes (1) definiert angeordnet ist.

21. Vorrichtung nach einem der Ansprüche 18 bis 20, bei der der Transmitter (21) von dem C-Bogen-Röntgengerät (1) oder der Patientenlagerungsvorrichtung (5) abnehmbar ist.

22. Vorrichtung nach einem der Ansprüche 17 bis 21, bei der wenigstens ein Positions- und Orientierungssensor (35) des elektromagnetischen Positionserfassungs- und Mappingsystems (2) an oder in der Patientenlagerungsvorrichtung (5) definiert angeordnet ist.

23. Vorrichtung nach Anspruch 22, bei der der wenigstens eine Positions- und Orientierungssensor (35) des elektromagnetischen Positionserfassungs- und Mappingsystems (2) von der Patientenlagerungsvorrichtung (5) abnehmbar ist.

24. Vorrichtung nach einem der Ansprüche 15 bis 23, bei der der Patient (P) oder ein Teil oder ein Organ des Patienten (P) mit Marken (6) versehen ist, deren Abbilder (16) in einem mit dem C-Bogen-Röntgengerät (1) gewonnenen Bild lokalisierbar sind und welche mit dem elektromagnetischen Positionserfassungs- und Mappingsystem (2) detektierbar sind.

25. Vorrichtung nach einem der Ansprüche 15 bis 24, bei der die Position des Patienten (P) während der Katheteranwendung ermittelt wird.

26. Vorrichtung nach einem der Ansprüche 15 bis 25, bei der die Bilddaten des mit dem C-Bogen-Röntgengerät (1) gewonnenen Bildes und die elektroanatomischen Mapping-Daten unter Berücksichtigung der Position des Patienten (P) bei der Erfassung des Bildes und der elektroanatomischen Mapping-Daten miteinander fusioniert oder einander überlagert werden.

27. Vorrichtung nach einem der Ansprüche 15 bis 26, bei der wenigstens ein Teil eines mit dem elektromagnetischen Positionserfassungs- und Mappingsystem (2) erfassbaren Katheters (3) oder eines mit dem elektromagnetischen Positionserfassungs- und Mappingsystem (2) erfassbaren Instrumentes unter Berücksichtigung der Position des Patienten (P) in das Bild des Patienten (P) und/oder die elektroanatomischen Mapping-Daten eingeblendet wird.

28. Vorrichtung nach einem der Ansprüche 15 bis 27, bei der der Patient (P) zur Erfassung der Position des Patienten (P) mit wenigstens einem Referenzsensor (22) des elektromagnetischen Positionserfassungs- und Mappingsystems (2) versehen ist.

29. Vorrichtung nach Anspruch 28, bei der die mit dem elektromagnetischen Positionserfassungs- und Mappingsystem (2) erfassten Positionen oder Koordinaten relativ zu dem Referenzsensor (22) des Patienten (P) erfasst werden.

## Claims

1. A method for visually assisting a catheter application on the heart (H) of a patient (P) using at least one image of the patient (P) obtained by means of a C-arm X-ray device (1) and using electroanatomical mapping data of the patient (P) that is obtained by means of an electromagnetic position-detection and mapping system (2), wherein the at least one image of the patient and the electroanatomical mapping data of the patient are merged together or superimposed on one another and wherein the C-arm X-ray device (1) and the electromagnetic position-detection and mapping system (2) are calibrated in relation to each other by means of the determination of a coordinate transformation between a coordinate system (CR) assigned to the C-arm X-ray device (1) and/or a coordinate system (CB) that is assigned to at least one image generated by the C-arm X-ray device (1) and a coordinate system (CM) assigned to the electromagnetic position-detection and mapping system (2), and wherein the position of the patient (P) is determined during the acquisition of the image and during the acquisition of the electroanatomical mapping data, which were obtained prior to the catheter application, and is at least indirectly assigned to the image and the electroanatomical mapping-data, wherein the C-arm X-ray device (1) assigns an identifier to an image that has been taken of the patient (P) at the time of acquisition of the image and transmits the identifier at the time of acquisition of the image to the electromagnetic position-detection and mapping system (2), the electromagnetic position-detection and mapping system (2) storing the position of the patient (P), including the identifier, at the time of acquisition of the image.

2. A method, especially according to claim 1, for visually assisting a catheter application on the heart (H) of a patient (P) using at least one image of the patient (P) obtained by means of a C-arm X-ray device (1) and using electroanatomical mapping data of the patient (P) that is obtained by means of an electromagnetic position-detection and mapping system (2), wherein the at least one image of the patient and the electroanatomical mapping data of the patient are merged together or superimposed on one another and wherein the C-arm X-ray device (1) and the electromagnetic position-detection and mapping system (2) are calibrated in relation to each other by means of the determination of a coordinate transformation between a coordinate system (CR) assigned to the C-arm X-ray device (1) and/or a coordinate system (CB) that is assigned to at least one image generated by the C-arm X-ray device (1) and a coordinate system (CM) assigned to the electromagnetic position-detection and mapping system (2), and wherein the position of the patient (P) is determined during the acquisition of the image and during the acquisition of the electroanatomical mapping data, which were obtained prior to the catheter application, and is at least indirectly assigned to the image and the electroanatomical mapping-data, wherein a time synchronization is achieved between the acquisition of an image of the patient (P) by the C-arm X-ray device (1) and the position of the patient (P), the time of acquisition being assigned to an image that has been taken and the position of the patient (P) being determined over time, so that the position of the patient (P) at the time of acquisition of the image can be determined by comparing the times.

3. The method according to claim 1 or 2, wherein the determination of the coordinate transformation between the coordinate system (CR) assigned to the C-arm X-ray device (1) and/or the coordinate system (CB) that is assigned to the at least one image generated by the C-arm X-ray device (1) and the coordinate system (CM) assigned to the electromagnetic position-detection and mapping system (2) is achieved using at least one marker (6, 25, 31), which is depicted in a generated image and is detectable using the electromagnetic position-detection and mapping system (2).

4. The method according to claim 3, wherein the marker (6, 25, 31) is an X-ray positive marker (6, 25, 31) and is depicted in at least two X-ray projections taken at projection angles that differ from each other or in a 3D image.

5. The method according to claim 4, wherein the image (16) of the marker (6) is located manually or automatically in the projection images or the 3D image using a method of pattern recognition.

6. The method according to any one of claims 3 to 5, wherein the marker (6, 31) is touched by a position sensor (3) of the electromagnetic position-detection and mapping system (2).

7. The method according to any one of claims 3 to 6, wherein at least one marker is an X-ray positive position sensor (25) of the electromagnetic position-detection and mapping system (2).

8. The method according to any one of claims 3 to 7, wherein a phantom (30) comprising at least one marker is (31) provided.

9. The method according to claim 8, wherein at least one marker of the phantom (30) is a position sensor of the electromagnetic position-detection and mapping system (2).

10. The method according to claim 9, wherein all the markers of the phantom (30) are position sensors of the electromagnetic position-detection and mapping system (2).

11. The method according to claim 9, wherein the phantom (30) comprises, in addition to the position sensor of the electromagnetic position-detection and mapping system (2), at least one further marker, the position of the at least one further marker of the phantom (30) relative to the at least one position sensor of the phantom (30) being known.

12. The method according to any one of claims 8 to 11, wherein the phantom (30) and the electromagnetic position-detection and mapping system (2) are arranged in a defined manner relative to each other such that the coordinate transformation between a coordinate system assigned to the phantom (30) and a coordinate system (CM) assigned to the electromagnetic position-detection and mapping system (2) is known.

13. The method according to any one of claims 1 to 12, wherein the C-arm X-ray device (1) retrieves the position of the patient (P) from the electromagnetic position-detection and mapping system (2) at the time of acquisition of an image of the patient (P) and assigns it to the image that has been acquired.

14. The method according to any one of claims 1 to 13, wherein a change in the position of the patient (P) relative to the image data that has been recorded is taken into account in the merging or superimposition of image data of an image of the patient (P) recorded using the C-arm X-ray device (1) before a catheter application with electroanatomical mapping data of the patient (P) recorded before or during a catheter application or in the navigation of a catheter (3) based on image data of an image of the patient (P) recorded using the C-arm X-ray device (1) before a catheter application and/or based on electroanatomical mapping data recorded before a catheter application.

15. A device for visually assisting a catheter application on the heart (H) of a patient (P) using at least one image of the patient (P) obtained by means of a C-arm X-ray device (1) and using electroanatomical mapping data of the patient (P) obtained by means of an electromagnetic position-detection and mapping system (2), wherein the at least one image of the patient and the electroanatomical mapping data of the patient are merged together or superimposed on one another, said device comprising a C-arm X-ray device (1), an electromagnetic position-detection and mapping system (2) and at least one computing device (10, 23, 24), which is adapted for the determination of a coordinate transformation between a coordinate system (CR) assigned to the C-arm X-ray device (1) and/or a coordinate system (CB) that is assigned to at least one image generated by the C-arm X-ray device (1) and a coordinate system (CM) assigned to the electromagnetic position-detection and mapping system (2), wherein the position of the patient (P) is determined during the acquisition of the image and during the acquisition of the electroanatomical mapping data, which were obtained prior to the catheter application, and is at least indirectly assigned to the image and electroanatomical mapping-data, wherein the C-arm X-ray device (1) assigns an identifier to an image that has been taken of the patient (P) at the time of acquisition of the image and transmits the identifier to the electromagnetic position-detection and mapping system (2) at the time of acquisition of the image, the electromagnetic position-detection and mapping system (2) storing the position of the patient (P), including the identifier, at the time of acquisition of the image.

16. A device, especially according to claim 15, for visually assisting a catheter application on the heart (H) of a patient (P) using at least one image of the patient (P) obtained by means of a C-arm X-ray device (1) and using electroanatomical mapping data of the patient (P) that is obtained by means of an electromagnetic position-detection and mapping system (2), wherein the at least one image of the patient and the electroanatomical mapping data of the patient are merged together or superimposed on one another, said device comprising a C-arm X-ray device (1), an electromagnetic position-detection and mapping system (2) and at least one computing device (10, 23, 24), which is adapted for the determination of a coordinate transformation between a coordinate system (CR) assigned to the C-arm X-ray device (1) and/or a coordinate system (CB) that is assigned to at least one image generated by the C-arm X-ray device (1) and a coordinate system (CM) assigned to the electromagnetic position-detection and mapping system (2), wherein the position of the patient (P) is determined during the acquisition of the image and during the acquisition of the electroanatomical mapping data, which were obtained prior to the catheter application, and is at least indirectly assigned to the image and electroanatomical mapping-data, wherein a time synchronization is achieved between the acquisition of an image of the patient (P) by the C-arm X-ray device (1) and the position of the patient (P), wherein the time of acquisition is assigned to an image that has been taken and the position of the patient (P) is detected over time, so that the position of the patient (P) at the time of acquisition of the image can be determined by comparing the times.

17. The device according to claim 15 or 16, wherein a patient supporting device (5) is assigned to the C-arm X-ray device (1) in a defined manner.

18. The device according to any one of claims 15 to 17, wherein the electromagnetic position-detection and mapping system (2) comprises at least one transmitter (21) to generate an electromagnetic alternating field and a catheter (3) comprising at least one sensor.

19. The device according to claim 18, wherein the transmitter (21) is arranged on the C-arm X-ray device (1) or on the patient supporting device (5) in a defined manner.

20. The device according to claim 18 or 19, wherein the transmitter (21) is arranged on the C-arm (7) of the C-arm X-ray device (1) in a defined manner.

21. The device according to any one of claims 18 to 20, wherein the transmitter (21) is detachable from the C-arm X-ray device (1) or the patient supporting device (5).

22. The device according to any one of claims 17 to 21, wherein at least one positioning and orientation sensor (35) of the electromagnetic position-detection and mapping system (2) is arranged on or in the patient supporting device (5) in a defined manner.

23. The device according to claim 22, wherein the at least one positioning and orientation sensor (35) of the electromagnetic position-detection and mapping system (2) is detachable from the patient supporting device (5).

24. The device according to any one of claims 15 to 23, wherein the patient (P) or a part or an organ of the patient (P) is provided with markers (6), the images of which (16) are locatable in an image obtained using the C-arm X-ray device and are detectable using the electromagnetic position-detection and mapping system (2).

25. The device according to any one of claims 15 to 24, wherein the position of the patient (P) is determined during the catheter application.

26. The device according to any one of claims 15 to 25, wherein, taking into account the position of the patient (P), the image data of the image obtained using the C-arm X-ray device (1) and the electroanatomical mapping data is merged or superimposed on one another during the acquisition of the image and of the electroanatomical mapping data,.

27. The device according to any one of claims 15 to 26, wherein at least part of a catheter (3) that is detectable using the electromagnetic position-detection and mapping system (2) or of an instrument that is detectable using the electromagnetic position-detection and mapping system (2) is blended into the image of the patient (P) and/or the electroanatomical mapping data, taking into account the position of the patient (P).

28. The device according to any one of claims 15 to 27, wherein the patient (P) is provided with at least one reference sensor (22) of the electromagnetic position-detection and mapping system (2) to detect the position of the patient (P).

29. The device according to claim 28, wherein the positions or coordinates detected using the electromagnetic position-detection and mapping system (2) are detected relative to the reference sensor (22) of the patient (P).

## Revendications

1. Procédé d'assistance visuelle pour l'application d'un cathéter sur le coeur (H) d'un patient (P) à l'aide d'au moins une image obtenue du patient (P) avec un appareil de radiographie par arceaux en C (1) et à l'aide de données cartographiques électro-anatomiques du patient (P) obtenues avec un système de cartographie et de détection de position électromagnétique (2), dans lequel l'au moins une image du patient et les données cartographiques électro-anatomiques du patient sont fusionnées l'une avec l'autre ou superposées l'une à l'autre et dans lequel l'appareil de radiographie par arceaux en C (1) et le système de cartographie et de détection de position électromagnétique (2) sont calibrés l'un par rapport à l'autre, par le fait qu'une transformation de coordonnées entre un système de coordonnées (CR) affecté à l'appareil de radiographie par arceaux en C (1) et/ou un système de coordonnées (CB) affecté à au moins une image générée avec l'appareil de radiographie par arceaux en C (1) et un système de coordonnées (CM) affecté au système de cartographie et de détection de position électromagnétique (2) est déterminée, et dans lequel la position du patient (P) pendant la capture de l'image et pendant la capture des données cartographiques électro-anatomiques, qui ont été obtenues avant l'application d'un cathéter, est déterminée et affectée au moins indirectement à l'image et aux données cartographiques électro-anatomiques, dans lequel l'appareil de radiographie par arceaux en C (1) affecte une marque d'identification à une image prise du patient (P) au moment de la capture de l'image et transmet la marque d'identification au moment de la capture de l'image au système de cartographie et de détection de position électromagnétique (2), dans lequel le système de cartographie et de détection de position électromagnétique (2) enregistre la position du patient (P) y compris la marque d'identification au moment de la capture de l'image.

2. Procédé, en particulier selon la revendication 1, d'assistance visuelle pour l'application d'un cathéter sur le coeur (H) d'un patient (P) à l'aide d'au moins une image obtenue du patient (P) avec un appareil de radiographie par arceaux en C (1) et à l'aide de données cartographiques électro-anatomiques du patient (P) obtenues avec un système de cartographie et de détection de position électromagnétique (2), dans lequel l'au moins une image du patient et les données cartographiques électro-anatomiques du patient sont fusionnées l'une avec l'autre ou superposées l'une à l'autre et dans lequel l'appareil de radiographie par arceaux en C (1) et le système de cartographie et de détection de position électromagnétique (2) sont calibrés l'un par rapport à l'autre, par le fait qu'une transformation de coordonnées entre un système de coordonnées (CR) affecté à l'appareil de radiographie par arceaux en C (1) et/ou un système de coordonnées (CB) affecté à au moins une image générée avec l'appareil de radiographie par arceaux en C (1) et un système de coordonnées (CM) affecté au système de cartographie et de détection de position électromagnétique (2) est déterminée, et dans lequel la position du patient (P) pendant la capture de l'image et pendant la capture des données cartographiques électro-anatomiques, qui ont été obtenues avant l'application d'un cathéter, est déterminée et affectée au moins indirectement à l'image et aux données cartographiques électro-anatomiques, dans lequel une synchronisation temporelle entre la capture d'une image du patient (P) avec l'appareil de radiographie par arceaux en C (1) et la position du patient (P) a lieu, dans lequel l'heure de capture est affectée à une image prise et la position du patient (P) est capturée au cours du temps, si bien que la position du patient (P) à l'heure de capture de l'image peut être déterminée par comparaison d'heure.

3. Procédé selon la revendication 1 ou 2, dans lequel la détermination de la transformation de coordonnées entre le système de coordonnées (CR) affecté à l'appareil de radiographie par arceaux en C (1) et/ou le système de coordonnées (CB) affecté à l'au moins une image générée avec l'appareil de radiographie par arceaux en C (1) et le système de coordonnées (CM) affecté au système de cartographie et de détection de position électromagnétique (2) a lieu à l'aide d'au moins un repère (6, 25, 31), qui est représenté dans une image générée et peut être détecté avec le système de cartographie et de détection de position électromagnétique (2).

4. Procédé selon la revendication 3, dans lequel le repère (6, 25, 31) est un repère radio-opaque (6, 25, 31) et est représenté dans au moins deux projections radiologiques prises en formant deux angles de projection différents l'un de l'autre ou dans une image 3D.

5. Procédé selon la revendication 4, dans lequel la représentation (16) du repère (6) dans les images de projection ou l'image 3D est localisée manuellement ou automatiquement avec un procédé de détection de motif.

6. Procédé selon l'une quelconque des revendications 3 à 5, dans lequel le repère (6, 31) est touché avec un capteur de position (3) du système de cartographie et de détection de position électromagnétique (2).

7. Procédé selon l'une quelconque des revendications 3 à 6, dans lequel l'au moins un repère est un capteur de position radio-opaque (25) du système de cartographie et de détection de position électromagnétique (2).

8. Procédé selon l'une quelconque des revendications 3 à 7, dans lequel un fantôme (30) doté d'au moins un repère (31) est prévu.

9. Procédé selon la revendication 8, dans lequel au moins un repère du fantôme (30) est un capteur de position du système de cartographie et de détection de position électromagnétique (2).

10. Procédé selon la revendication 9, dans lequel toutes les marques du fantôme (30) sont des capteurs de position du système de cartographie et de détection de position électromagnétique (2).

11. Procédé selon la revendication 9, dans lequel le fantôme (30) présente en plus du capteur de position du système de cartographie et de détection de position électromagnétique (2) au moins un autre repère, dans lequel la position de l'au moins un autre repère du fantôme (30) par rapport à l'au moins un capteur de position du fantôme (30) est connue.

12. Procédé selon l'une quelconque des revendications 8 à 11, dans lequel le fantôme (30) et le système de cartographie et de détection de position électromagnétique (2) sont agencés de manière définie l'un par rapport à l'autre de sorte que la transformation de coordonnées entre un système de coordonnées affecté au fantôme (30) et un système de coordonnées (CM) affecté au système de cartographie et de détection de position électromagnétique (2) est connue.

13. Procédé selon l'une quelconque des revendications 1 à 12, dans lequel l'appareil de radiographie par arceaux en C (1) appelle la position du patient (P) au moment de la capture d'une image du patient (P) par le système de cartographie et de détection de position électromagnétique (2) et l'affecte à l'image capturée.

14. Procédé selon l'une quelconque des revendications 1 à 13, dans lequel lors d'une fusion ou superposition de données image d'une image du patient (P) prises avec l'appareil de radiographie par arceaux en C (1) avant l'application d'un cathéter avec des données cartographiques électro-anatomiques du patient (P) prises avant ou pendant l'application d'un cathéter ou lors d'une navigation d'un cathéter (3) sur la base de données image d'une image du patient (P) prises avec l'appareil de radiographie par arceaux en C (1) avant l'application d'un cathéter et/ou sur la base de données cartographiques électro-anatomiques prises avant l'application d'un cathéter, un changement de la position du patient (P) est prise en compte par rapport aux données image prises.

15. Dispositif d'assistance visuelle pour l'application d'un cathéter sur le coeur (H) d'un patient (P) à l'aide d'au moins une image obtenue du patient (P) avec un appareil de radiographie par arceaux en C (1) et à l'aide de données cartographiques électro-anatomiques du patient (P) obtenues avec un système de cartographie et de détection de position électromagnétique (2), dans lequel l'au moins une image du patient et les données cartographiques électro-anatomiques du patient sont fusionnées l'une avec l'autre ou superposées l'une à l'autre, présentant un appareil de radiographie par arceaux en C (1), un système de cartographie et de détection de position électromagnétique (2) et au moins un dispositif de calcul (10, 23, 24), qui est conçu pour la détermination d'une transformation de coordonnées entre un système de coordonnées (CR) affecté à l'appareil de radiographie par arceaux en C (1) et/ou un système de coordonnées (CB) affecté à au moins une image générée avec l'appareil de radiographie par arceaux en C (1) et un système de coordonnées (CM) affecté au système de cartographie et de détection de position électromagnétique (2), et dans lequel la position du patient (P) pendant la capture de l'image et pendant la capture des données cartographiques électro-anatomiques, qui ont été obtenues avant l'application d'un cathéter, est déterminée et affectée au moins indirectement à l'image et aux données cartographiques électro-anatomiques, dans lequel l'appareil de radiographie par arceaux en C (1) affecte une marque d'identification à une image prise du patient (P) au moment de la capture de l'image et transmet la marque d'identification au moment de la capture de l'image au système de cartographie et de détection de position électromagnétique (2), dans lequel le système de cartographie et de détection de position électromagnétique (2) enregistre la position du patient (P) y compris la marque d'identification au moment de la capture de l'image.

16. Dispositif, en particulier selon la revendication 15, d'assistance visuelle pour l'application d'un cathéter sur le coeur (H) d'un patient (P) à l'aide d'au moins une image obtenue du patient (P) avec un appareil de radiographie par arceaux en C (1) et à l'aide de données cartographiques électro-anatomiques du patient (P) obtenues avec un système de cartographie et de détection de position électromagnétique (2), dans lequel l'au moins une image du patient et les données cartographiques électro-anatomiques du patient sont fusionnées l'une avec l'autre ou superposées l'une à l'autre, présentant un appareil de radiographie par arceaux en C (1), un système de cartographie et de détection de position électromagnétique (2) et au moins un dispositif de calcul (10, 23, 24), qui est conçu pour la détermination d'une transformation de coordonnées entre un système de coordonnées (CR) affecté à l'appareil de radiographie par arceaux en C (1) et/ou un système de coordonnées (CB) affecté à au moins une image générée avec l'appareil de radiographie par arceaux en C (1) et un système de coordonnées (CM) affecté au système de cartographie et de détection de position électromagnétique (2), dans lequel la position du patient (P) pendant la capture de l'image et pendant la capture des données cartographiques électro-anatomiques, qui ont été obtenues avant l'application d'un cathéter, est déterminée et affectée au moins indirectement à l'image et aux données cartographiques électro-anatomiques, dans lequel une synchronisation temporelle entre la capture d'une image du patient (P) avec l'appareil de radiographie par arceaux en C (1) et la position du patient (P) a lieu, dans lequel l'heure de capture est affectée à une image prise et la position du patient (P) est capturée au cours du temps, si bien que la position du patient (P) à l'heure de capture de l'image peut être déterminée par comparaison d'heure.

17. Dispositif selon la revendication 15 ou 16, dans lequel un dispositif de positionnement du patient (5) est affecté de manière définie à l'appareil de radiographie par arceaux en C (1).

18. Dispositif selon l'une quelconque des revendications 15 à 17, dans lequel le système de cartographie et de détection de position électromagnétique (2) présente au moins un transmetteur (21) pour la génération d'un champ alternatif électromagnétique et un cathéter (3) avec au moins un capteur.

19. Procédé selon la revendication 18, dans lequel le transmetteur (21) est agencé de manière définie au niveau de l'appareil de radiographie par arceaux en C (1) ou du dispositif de positionnement du patient (5).

20. Dispositif selon la revendication 18 ou 19, dans lequel le transmetteur (21) est agencé de manière définie au niveau de l'arc C (7) de l'appareil de radiographie par arceaux en C (1).

21. Dispositif selon l'une quelconque des revendications 18 à 20, dans lequel le transmetteur (21) peut être retiré de l'appareil de radiographie par arceaux en C (1) ou du dispositif de positionnement du patient (5).

22. Dispositif selon l'une quelconque des revendications 17 à 21, dans lequel au moins un capteur de position et d'orientation (35) du système de cartographie et de détection de position électromagnétique (2) est agencé de manière définie au niveau de ou dans le dispositif de positionnement du patient (5).

23. Dispositif selon la revendication 22, dans lequel l'au moins un capteur de position et d'orientation (35) du système de cartographie et de détection de position électromagnétique (2) peut être retiré du dispositif de positionnement du patient (5).

24. Dispositif selon l'une quelconque des revendications 15 à 23, dans lequel le patient (P) ou une partie ou un organe du patient (P) est doté de repères (6), dont les représentations (16) peuvent être localisées dans une image obtenue avec l'appareil de radiographie par arceaux en C (1) et qui peuvent être détectées avec le système de cartographie et de détection de position électromagnétique (2).

25. Dispositif selon l'une quelconque des revendications 15 à 24, dans lequel la position du patient (P) est déterminée pendant l'application d'un cathéter.

26. Dispositif selon l'une quelconque des revendications 15 à 25, dans lequel les données image de l'image obtenue avec l'appareil de radiographie par arceaux en C (1) et les données cartographiques électro-anatomiques sont fusionnées l'une avec l'autre ou superposées l'une à l'autre en tenant compte de la position du patient (P) lors de la détection de l'image et des données cartographiques électro-anatomiques.

27. Dispositif selon l'une quelconque des revendications 15 à 26, dans lequel au moins une partie d'un cathéter (3) pouvant être détecté avec le système de cartographie et de détection de position électromagnétique (2) ou d'un instrument pouvant être détecté avec le système de cartographie et de détection de position électromagnétique (2) est affichée en tenant compte de la position du patient (P) dans l'image du patient (P) et/ou dans les données cartographiques électro-anatomiques.

28. Dispositif selon l'une quelconque des revendications 15 à 27, dans lequel le patient (P) est doté d'au moins un capteur de référence (22) du système de cartographie et de détection de position électromagnétique (2) pour la détection de la position du patient (P).

29. Dispositif selon la revendication 28, dans lequel les positions ou coordonnées détectées avec le système de cartographie et de détection de position électromagnétique (2) sont détectées par rapport au capteur de référence (22) du patient (P).
